(19) Europäisches Patentamt
European Patent Office
Office européen des brevets

(11) **EP 2 767 210 A1**

## (12) EUROPEAN PATENT APPLICATION
published in accordance with Art. 153(4) EPC

(43) Date of publication:
**20.08.2014 Bulletin 2014/34**

(21) Application number: **12839296.6**

(22) Date of filing: **11.10.2012**

(51) Int Cl.:
**A61B 1/00** (2006.01)

(86) International application number:
**PCT/JP2012/076291**

(87) International publication number:
**WO 2013/054835 (18.04.2013 Gazette 2013/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.10.2011   JP 2011225143
04.09.2012   JP 2012193908**

(71) Applicant: **Fujifilm Corporation
Minato-ku
Tokyo 106-8620 (JP)**

(72) Inventors:
• **YAMAGUCHI, Hiroshi
Ashigarakami-gun
Kanagawa 258-8538 (JP)**

• **OZAWA, Satoshi
Ashigarakami-gun
Kanagawa 258-8538 (JP)**
• **IIDA, Takayuki
Ashigarakami-gun
Kanagawa 258-8538 (JP)**

(74) Representative: **Klunker . Schmitt-Nilson . Hirsch
Patentanwälte
Destouchesstraße 68
80796 München (DE)**

## (54) ENDOSCOPE SYSTEM AND IMAGE GENERATION METHOD

(57) The visibility of irregularities on the body tissue, such as a superficial microstructure or hypertrophy, is improved. Excitation light EL is emitted to a phosphor to excite and emit white light W. High absorption wavelength cut light is generated by removing components in high absorption wavelength bands A1 and A2, in which the absorption coefficient of hemoglobin in the blood is high, from the white light using a high absorption wavelength rejection filter. The subject is illuminated with the high absorption wavelength cut light, and image light of the reflected light is captured by a color CCD. A microstructure image is generated based on a signal Bp output from the B pixel of the CCD. In this microstructure image, the display of superficial microvessels is suppressed. Accordingly, the visibility of superficial microstructures, such as a pit pattern, is relatively improved.

FIG. 4

Description

BACKGROUND OF THE INVENTION

1. Field of the Invention

The present invention relates to an endoscope system capable of clearly observing a microstructure such as a pit pattern or an irregular pattern such as hypertrophy, which is formed on body tissue, and an image generation method.

2. Description of the Related Art

In recent medical treatment, diagnosis or the like using an endoscope apparatus has been widely performed. In this endoscopic diagnosis, not only normal light observation, in which white light of broadband light is used as illumination light within the subject, but also special light observation, in which a lesion, such as cancer, is made clearer than other parts or the position or the size of the lesion is easily intuitively grasped by using the special light having a specific wavelength as illumination light, is performed.

For example, in JP2001-170009A, using the fact that the degree of penetration in the depth direction of the body tissue and the absorption characteristics of hemoglobin in the blood have a wavelength dependency, a microstructure such as a pit pattern or a microvessel formed in a body tissue surface layer is made clear with blue narrow-band light having a short wavelength, and a thick blood vessel located in a medium-deep layer of the body tissue is made clear with green narrow-band light having a longer wavelength than that of the blue narrow-band light. Blood vessels or superficial microstructures of the surface to medium-deep layers are important clues at the time of differential diagnosis of cancer or degree-of-penetration diagnosis. Therefore, it is possible to greatly improve the accuracy of differentiation and the like by making the blood vessels or the superficial microstructures of the surface to medium-deep layers clear using blue narrow-band light or green narrow-band light.

In addition, in JP1996-252218A (JP-H08-252218A), a boundary between a lesion part and a normal part is made clear by using the characteristic that the amount of auto-fluorescence emitted from the lesion part, which is thickened due to the lesion such as cancer, is less than the amount of auto-fluorescence from the normal part, which is not thickened, when irradiating the body tissue with excitation light for exciting the auto-fluorescence. By making the boundary between the lesion part and the normal part clear as described above, it becomes easy to grasp the position or the size of the lesion part when performing observation from a distant-view state as at the time of screening.

SUMMARY OF THE INVENTION

In recent years, there are various kinds of cancer differentiation methods or methods for degree-of-penetration diagnosis. Accordingly, there is not only a case where cancer diagnosis is performed from both a blood vessel pattern, such as a superficial microvessel or a medium-deep layer blood vessel, and an irregular pattern, such as a superficial microstructure or hypertrophy, but also a case where diagnosis is performed by focusing only on the irregular pattern. When performing diagnosis by focusing only on the irregular pattern as described above, it is necessary to reduce the visibility of the blood vessel pattern while improving the visibility of the irregular pattern.

For making only the irregular pattern clear, there is no description and suggestion in JP2001-170009A. In addition, according to JP1996-252218A (JP-H08-252218A), it is possible to make the hypertrophy of the irregular pattern clear. However, auto-fluorescence used to detect the hypertrophy is weak. Therefore, in order to capture the auto-fluorescence with good sensitivity, a high-sensitivity imaging device such as an EMCCD is separately required.

It is an object of the present invention to provide an endoscope system and an image generation method capable of improving the visibility of irregularities on body tissue, such as a superficial microstructure or a hypertrophy.

An endoscope system of the present invention includes: an illumination means for irradiating a subject with illumination light; an image signal acquisition means for acquiring an image signal by capturing image light of reflected light from the subject; and an irregularity image generation means for generating an irregularity image, in which visibility of irregularities on body tissue is relatively improved, by suppressing display of blood vessels in the subject based on the image signal.

Preferably, the irregularity image generation means includes a microstructure image generation section that generates a microstructure image, in which visibility of superficial microstructures is relatively improved, by suppressing display of blood vessels based on a signal obtained by removing a component of a first high absorption wavelength band, in which an absorption coefficient of hemoglobin in blood is high, in a blue wavelength band from the image signal. It is preferable that the first high absorption wavelength band be 400 nm to 450 nm.

Preferably, the irregularity image generation means includes a microstructure image generation section that generates a microstructure image, in which visibility of hypertrophy is relatively improved, by suppressing display of blood vessels based on a signal obtained by removing a component of a second high absorption wavelength band, in which an absorption coefficient of hemoglobin in blood is high, in a green wavelength band from the image signal. It is preferable that the second high absorption wavelength band be 520 nm to 580 nm.

In addition, in the present invention, preferably, the illumination means includes a high absorption wavelength rejection filter that removes a component of a high absorption wavelength band, in which an absorption coefficient of hemoglobin in blood is high, from the illumination light, and the image signal acquisition means captures image light of reflected light from the subject, from which the component of the high absorption wavelength band has been removed by the high absorption wavelength rejection filter. Preferably, imaging of the subject is performed by a color imaging device having pixels of a plurality of colors for which respective color separation filters are provided. Preferably, the illumination means irradiates the subject sequentially with light beams of a plurality of colors, and imaging of the subject is performed by a monochrome imaging device whenever the light beams of the plurality of colors are sequentially irradiated to the subject.

Preferably, the illumination means sequentially irradiates illumination light for a surface layer obtained by removing a component of a first high absorption wavelength band, in which an absorption coefficient of hemoglobin in blood is high, in a blue wavelength band and illumination light for a medium-deep layer obtained by removing a component of a first high absorption wavelength band, in which an absorption coefficient of hemoglobin in blood is high, in a green wavelength band, and imaging of the subject is performed whenever sequential irradiation is performed by the illumination means.

Preferably, the irregularity image generation means includes an image generation section that acquires a spectral image of a wavelength component other than a wavelength component, in which an absorption coefficient of hemoglobin in blood is high, of the reflected light by spectral estimation based on the image signal and generates the irregularity image based on the spectral image. It is preferable to further include display means for displaying the irregularity image.

An image generation method of the present invention includes: irradiating a subject with illumination light using illumination means; acquiring an image signal by capturing image light of reflected light from the subject using image signal acquisition means; and generating an irregularity image, in which visibility of irregularities on body tissue is relatively improved, by suppressing display of blood vessels in the subject based on the image signal using irregularity image generation means.

According to the present invention, in the irregularity image acquired by the irregularity image generation means, the visibility of irregularities on the body tissue is relatively improved by suppressing the display of blood vessels in the subject.

**BRIEF DESCRIPTION OF THE DRAWINGS**

Fig. 1 is a diagram showing an endoscope system of a first embodiment.
Fig. 2 is a diagram showing the internal configuration of the endoscope system of the first embodiment.
Fig. 3 is a graph showing the emission spectrum of white light W.
Fig. 4 is a graph showing the spectral transmittance of a high absorption wavelength rejection filter and the absorption coefficient of hemoglobin.
Fig. 5A is a diagram showing an image of a subject when a zoom lens is at a wide-angle position.
Fig. 5B is a diagram showing an image of a subject when a zoom lens is at a telephoto position.
Fig. 6A is a diagram showing B, G, and R pixels of a CCD.
Fig. 6B is a graph showing the spectral transmittances of color filters of R, G, and B colors.
Fig. 7A is a graph showing a wavelength component of light received by the B pixel of the CCD.
Fig. 7B is a graph showing a wavelength component of light received by the G pixel of the CCD.
Fig. 7C is a graph showing a wavelength component of light received by the R pixel of the CCD.
Fig. 8A is a diagram for explaining the imaging control of the CCD in a normal observation mode in the first embodiment.
Fig. 8B is a diagram for explaining the imaging control of the CCD in a microstructure observation mode, a hypertrophy observation mode, and a microstructure and hypertrophy mode in the first embodiment.
Fig. 9 is a diagram showing a microstructure image.
Fig. 10 is a diagram showing a hypertrophy image.
Fig. 11 is a flowchart showing a sequential flow in the microstructure observation mode.
Fig. 12 is a diagram showing the internal configuration of an endoscope system that generates the white light W using a xenon lamp.
Fig. 13 is a diagram showing the internal configuration of an endoscope system of a second embodiment.
Fig. 14 is a diagram showing an RGB rotary filter.
Fig. 15 is a graph showing the spectral transmittances of B, G, and R filters of the RGB rotary filter.
Fig. 16A is a diagram for explaining the imaging control of the CCD in a normal observation mode in the second embodiment.
Fig. 16B is a diagram for explaining the imaging control of the CCD in a microstructure observation mode, a hypertrophy observation mode, and a microstructure and hypertrophy mode in the second embodiment.
Fig. 17 is a diagram showing the internal configuration of an endoscope system of a third embodiment.
Fig. 18 is a diagram of a rotary filter for special observation.
Fig. 19 is a graph showing the spectral transmittance of a first BPF.
Fig. 20 is a graph showing the spectral transmittance of a second BPF.

Fig. 21A is a diagram for explaining the imaging control of the CCD in a normal observation mode in a third embodiment.

Fig. 21B is a diagram for explaining the imaging control of the CCD in a microstructure observation mode in the third embodiment.

Fig. 21C is a diagram for explaining the imaging control of the CCD in a hypertrophy observation mode in the third embodiment.

Fig. 21D is a diagram for explaining the imaging control of the CCD in a microstructure and hypertrophy mode in the third embodiment.

Fig. 22 is a diagram showing the internal configuration of an endoscope system of a fourth embodiment.

Fig. 23 is a diagram for explaining a method of generating a microstructure image.

Fig. 24 is a diagram for explaining a method of generating a hypertrophy image.

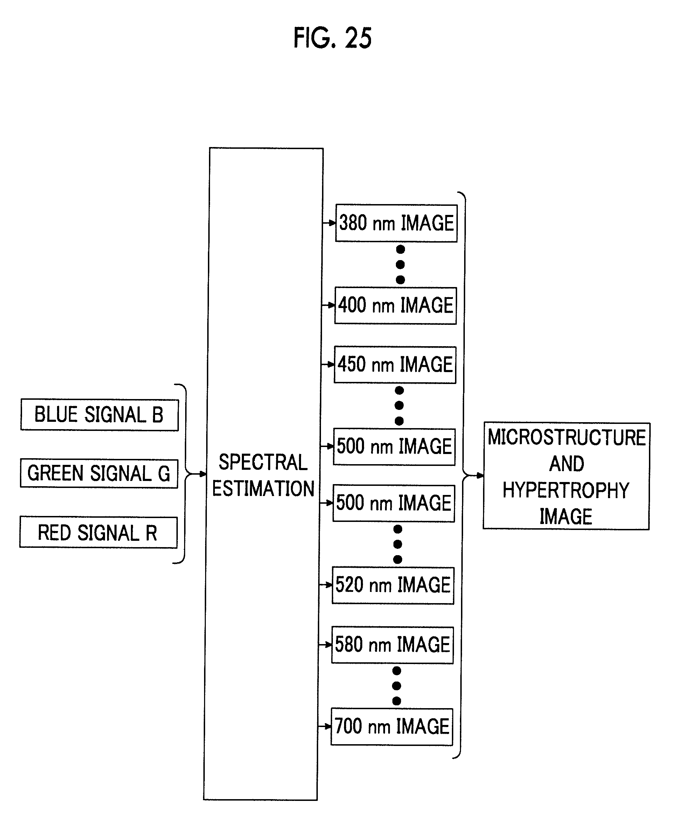

Fig. 25 is a diagram for explaining a method of generating a microstructure and hypertrophy image.

## DESCRIPTION OF THE PREFERRED EMBODIMENTS

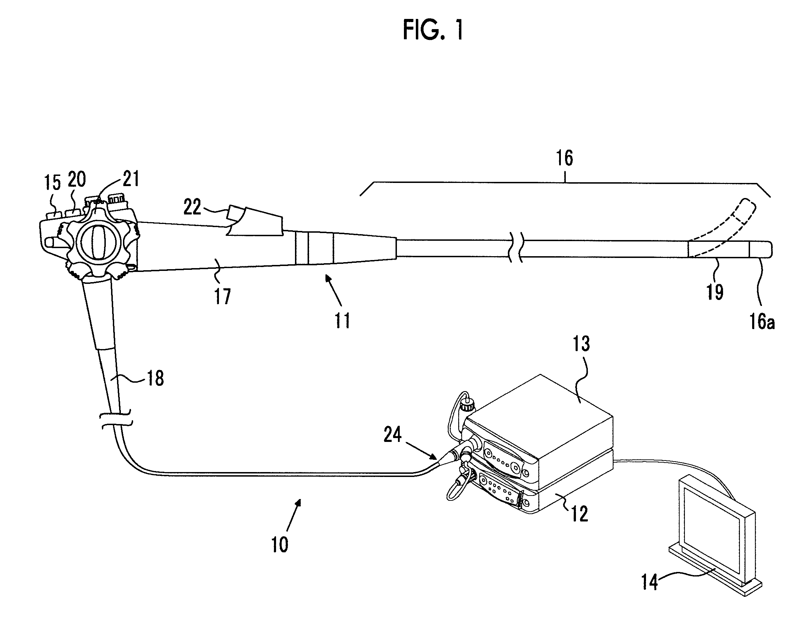

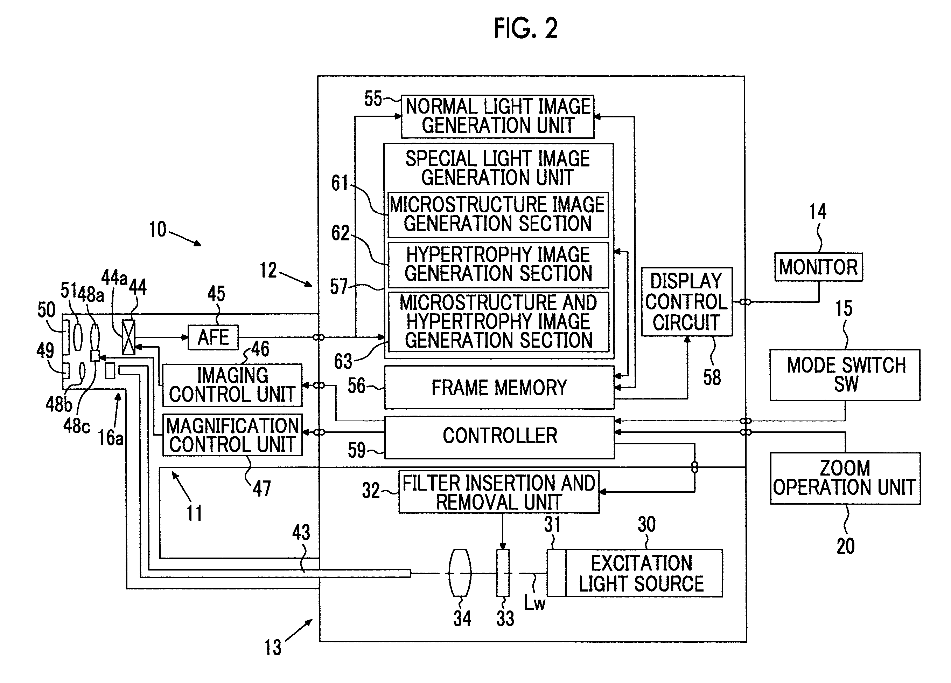

As shown in Figs. 1 and 2, an endoscope system 10 of a first embodiment includes: an electronic endoscope 11 (a form of image signal acquisition means) that images the inside of a subject; a processor device 12 that performs various kinds of image processing on the image captured by the electronic endoscope 11; a light source device 13 that supplies light for illuminating the subject to the electronic endoscope 11; and a monitor 14 that displays an image after various kinds of image processing are performed by the processor device 12.

The electronic endoscope 11 includes a flexible insertion unit 16 that is inserted into the subject, an operating unit 17 provided at the proximal end of the insertion unit 16, and a universal code 18 that makes a connection between the operating unit 17 and the processor device 12 and the light source device 13. A curved portion 19 obtained by connecting a plurality of curved pieces is formed at the distal end of the insertion unit 16. The curved portion 19 is curved in the horizontal and vertical directions by operating an angle knob 21 of the operating unit 17. A distal portion 16a including an optical system for imaging the body cavity and the like is provided at the distal end of the curved portion 19. The distal portion 16a is directed in a desired direction within the subject by the bending operation of the curved portion 19.

In addition, a mode switch SW 15 for switching to various modes is provided in the operating unit 17. The modes include a total of four modes of a normal observation mode in which a normal light image obtained by imaging a subject illuminated with white light is displayed on the monitor 14, a microstructure observation mode in which a microstructure enhancement image emphasizing the microstructure formed on the surface layer of body tissue is displayed on the monitor 14, a hypertrophy observation mode in which a hypertrophy enhancement image that emphasizes a hypertrophy having a thickness from the surface layer to the medium-deep layer in body tissue is displayed, and a microstructure and hypertrophy observation mode in which a microstructure and hypertrophy enhancement image emphasizing both the microstructure and the hypertrophy is displayed on the monitor 14.

A connector 24 is attached to the universal code 18 on the side of the processor device 12 and the light source device 13. The connector 24 is a composite connector including a communication connector and a light source connector, and the electronic endoscope 11 is detachably connected to the processor device 12 and the light source device 13 through the connector 24.

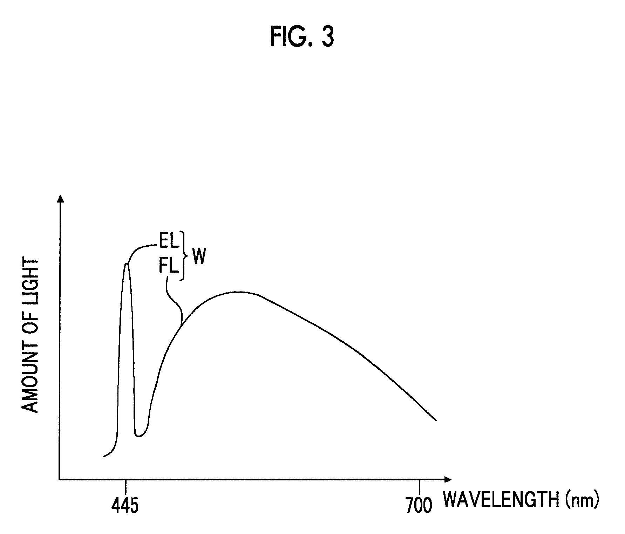

As shown in Fig. 2, the light source device 13 (a form of illumination means) includes an excitation light source 30, a phosphor 31, a filter insertion and removal unit 32, and a high absorption wavelength rejection filter 33. The excitation light source 30 is a semiconductor light source, such as a laser diode, and emits the excitation light EL having a center wavelength of 445 nm as shown in Fig. 3. The excitation light EL is irradiated onto the phosphor 31 attached to an emission section of the excitation light source 30. The phosphor 31 is configured to include a plurality of kinds of fluorescent materials (for example, a YAG-based fluorescent material or a fluorescent material, such as BAM ($BaMgAl_{10}O_{17}$)) that absorb a part of the excitation light EL and excite and emit fluorescence FL of green to red. The fluorescence FL excited and emitted by the phosphor 31 is combined with the excitation light EL that is transmitted through the phosphor 31 without being absorbed by the phosphor 31, thereby generating white light W.

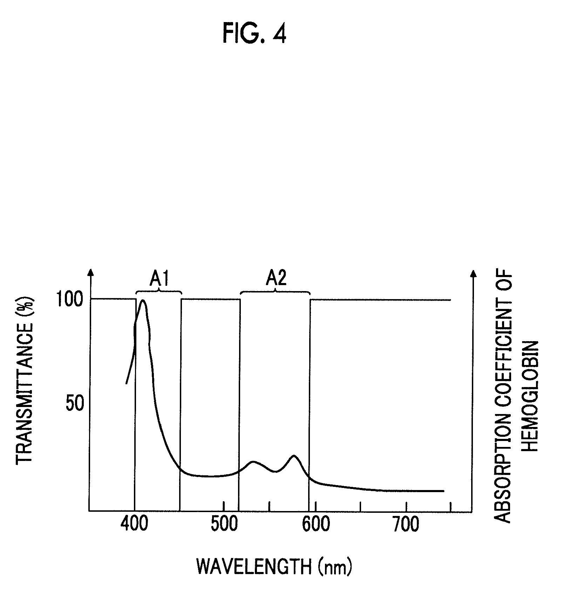

The filter insertion and removal unit 32 moves the high absorption wavelength rejection filter 33 between an insertion position, at which the high absorption wavelength rejection filter 33 is inserted in the optical path Lw of the white light W, and a retraction position, at which the high absorption wavelength rejection filter 33 is retracted from the optical path Lw, according to the mode that is set. When the normal observation mode is set, the high absorption wavelength rejection filter 33 is set at the retraction position. Accordingly, the white light W is incident on the light guide 43 through a condensing lens 34. On the other hand, when the microstructure observation mode, the hypertrophy observation mode, and the microstructure and hypertrophy observation mode are set, the high absorption wavelength rejection filter 33 is set at the insertion position. Therefore, high absorption wavelength cut light Wcut obtained by cutting light in a wavelength band (refer to Fig. 4), in which the absorption coefficient of hemoglobin is high, from the white light W, is transmitted through the high absorption wavelength rejection filter 33. The transmitted high absorption wavelength cut light Wcut is incident on the light guide 43 through the condensing lens 34.

As shown in Fig. 4, the high absorption wavelength rejection filter 32 cuts light in a high absorption wavelength band A1 of 400 nm to 450 nm and a high absorption wavelength band A2 of 520 nm to 580 nm (with a transmittance of 0%), and transmits light in a wavelength band other than the high absorption wavelength bands A1 and A2 (with a transmittance of 100%). When the white light W is incident on the high absorption wavelength rejection filter 32, the high absorption wavelength cut light Wcut obtained by removing the high absorption wavelengths A1 and A2 from the white light W is emitted from the high absorption wavelength rejection filter 32.

The reason why the light in the high absorption wavelength bands A1 and A2 is cut as described above is as follows. As shown in Fig. 4, the light in the high absorption wavelength bands A1 and A2 shows high absorption characteristics of hemoglobin in the blood. For this reason, when an image based on the image light of the high absorption wavelength bands A1 and A2 is displayed on the monitor 14, the contrast between blood vessels and other tissues is high. As a result, blood vessels are highlighted. Accordingly, when performing diagnosis by focusing not on the blood vessels but only on the superficial microstructure, such as a pit pattern, or irregularities, such as a hypertrophy having a thickness from a body tissue surface layer to a medium-deep layer, the clarity of blood vessels is one of the causes of reducing the diagnostic performance. Therefore, the display of blood vessels when displayed on the monitor 14 is suppressed by cutting the light in the high absorption wavelength bands A1 and A2, which has high absorption characteristics, of the reflected light of the white light W using the high absorption wavelength rejection filter 32. By suppressing the clarity of blood vessels as described above, the visibility of irregularities on the body tissue, such as a hypertrophy or a superficial microstructure, other than the blood vessels is improved.

As shown in Fig. 2, the electronic endoscope 11 includes the light guide 43, a CCD 44, an analog processing circuit 45 (AFE: Analog Front End), an imaging control unit 46, and a magnification control unit 47. The light guide 43 is a large-diameter optical fiber, a bundle fiber, or the like, and the incidence end is connected to the inside of the light source device and the exit end is directed to a zoom lens 48a. Accordingly, light guided by the light guide 43 is irradiated to the subject through an irradiation lens 48b and an illumination window 49. An observation window 50 receives reflected light from the subject. The received light is incident on the CCD 44 through a condensing lens 51 and a zoom lens 48a.

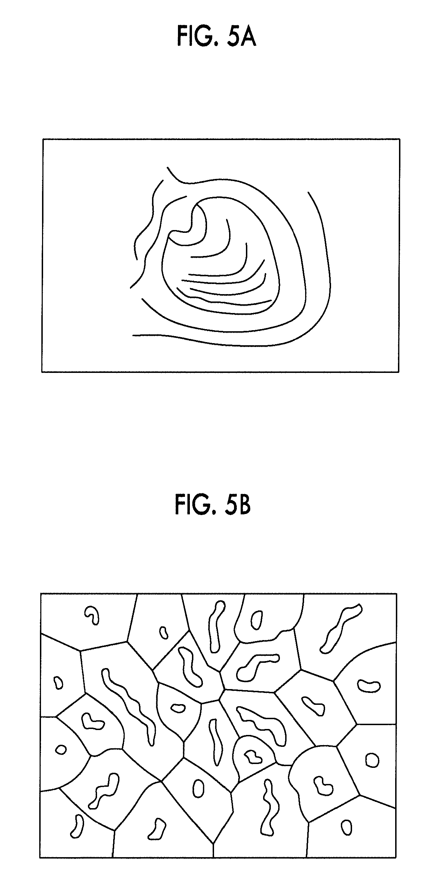

An actuator 48c to move the zoom lens 48a in the optical axis direction is attached to the zoom lens 48a. The driving of the actuator 48c is controlled by the magnification control unit 47 connected to the controller 59. The magnification control unit 47 controls the actuator 48c so that the zoom lens 48a moves to a position corresponding to the magnification set by a zoom operation unit 20. When it is necessary to observe the overall condition in the subject, for example, at the time of screening, the zoom lens 48a is set to a wide-angle position so that a non-enlarged image shown in Fig. 5A is displayed on the monitor 14. On the other hand, when it is necessary to observe the detailed structure of a part to be observed, for example, at the time of differential diagnosis of cancer, the zoom lens 48a is set to a telephoto position so that an enlarged image shown in Fig. 5B is displayed on the monitor 14.

In the normal observation mode and the hypertrophy observation mode, the overall condition in the subject is observed in many cases. Therefore, the zoom lens 48a is set to the wide-angle position in many cases. On the other hand, in the microstructure observation mode, an object to be observed is enlarged and observed in many cases. Therefore, the zoom lens 48a is set to the telephoto position in many cases.

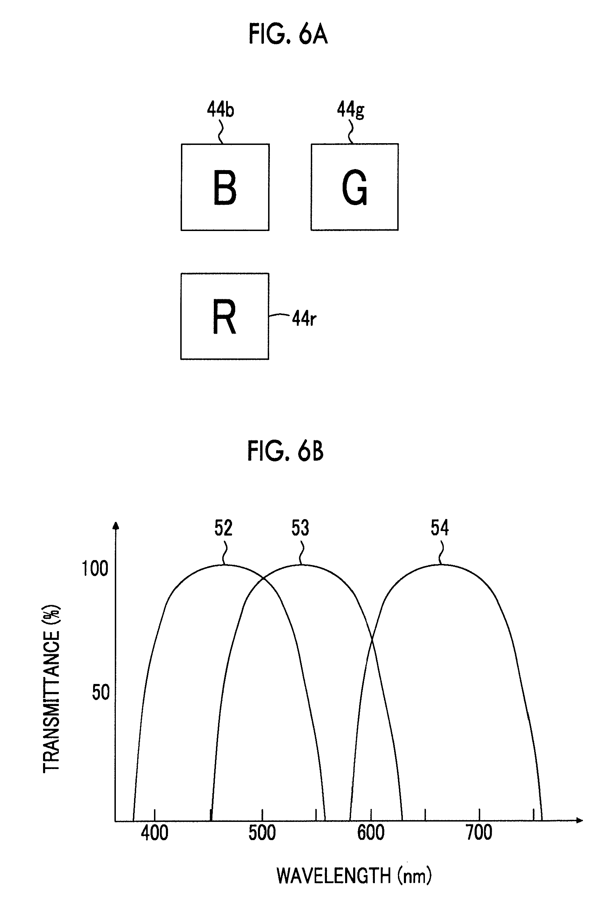

The CCD 44 has an imaging surface 44a to receive incident light, and performs photoelectric conversion on the imaging surface 44a and accumulates the signal charges. The accumulated signal charges are read as an imaging signal, and the imaging signal is transmitted to the AFE 45. The CCD 44 is a color CCD, and many pixels of three colors of a B pixel in which a B filter 44b of B color is provided, a G pixel in which a G filter 44g of G color is provided, and an R pixel in which an R filter 44r of R color is provided are arrayed on the imaging surface 44a, as shown in Fig. 6A. The B filter 44b, the G filter 44g, and the R filter 44r have B, G, and R transmission regions 52, 53, and 54, as shown in Fig. 6B. The B transmission region 52 occupies a wavelength range of 380 nm to 560 nm, the G transmission region 53 occupies a wavelength range of 450 nm to 630 nm, and the R transmission region 54 occupies a wavelength range of 580 nm to 780 nm.

Since the light received by the CCD 44 changes with a mode that is set, wavelength components received by the filters 44b, 44g, and 44r are also different. When the normal observation mode is set, the white light W is incident on the pixel of each color of the CCD 44. Accordingly, a wavelength component of the white light W included in the B transmission region 52 is incident on the B pixel, a wavelength component of the white light W included in the G transmission region 53 is incident on the G pixel, and a wavelength component of the white light W included in the R transmission region 54 is incident on the R pixel.

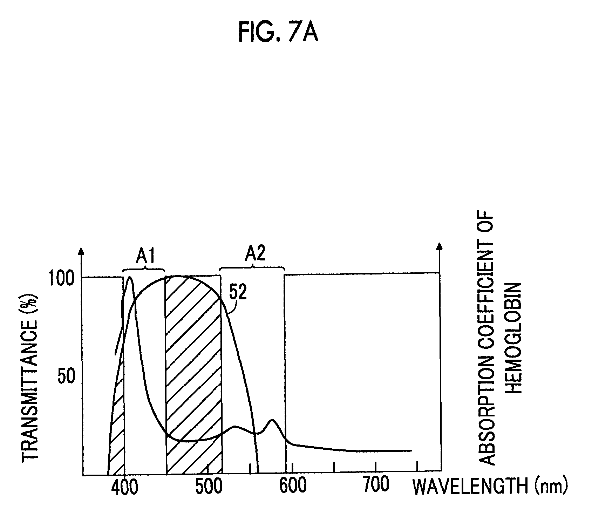

On the other hand, when the microstructure observation mode, the hypertrophy observation mode, and the microstructure and hypertrophy observation mode are set, the high absorption wavelength cut light Wcut is incident on the pixel of each color of the CCD 44. Accordingly, as shown in Fig. 7A, first transmitted light of 380 nm to 400 nm and 450 nm to 500 nm, which is included in the B transmission region 52, of the high absorption wavelength cut light Wcut is incident on the B pixel of the CCD 44. The first transmitted light has a degree of penetration to the body tissue surface layer, and the absorption characteristics of hemoglobin are low compared with the high absorption wavelength band A1 of 400 nm to 450 nm. Accordingly, when a captured image of image light of the first transmitted light is displayed on the monitor 14, the display of superficial microvessels is suppressed, while the visibility of the superficial microstructure, such as a pit pattern, is improved by suppressing the display of the blood vessels.

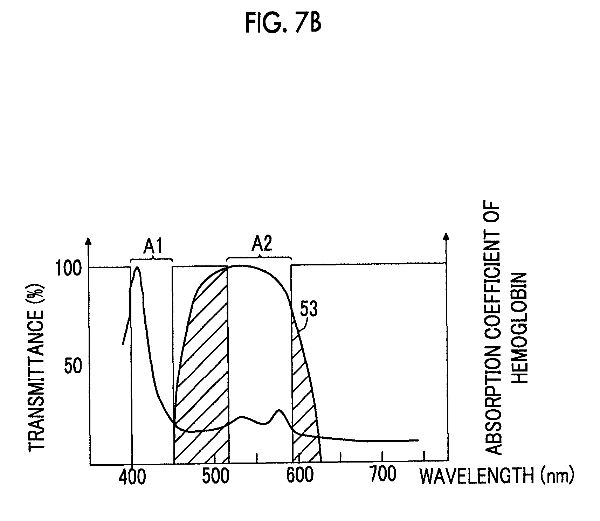

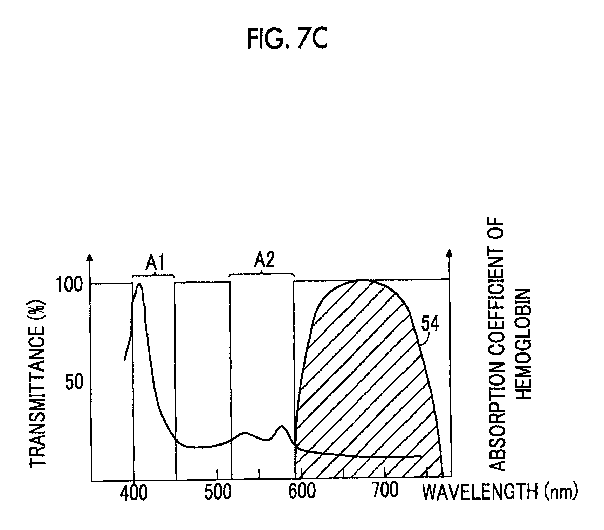

As shown in Fig. 7B, second transmitted light of 450 nm to 520 nm and 580 nm to 630 nm, which is included in the G transmission region, of the high absorption wavelength cut light Wcut is incident on the G pixel of the CCD 44. The second transmitted light has a degree of penetration to the medium-deep layer of body tissue, and the absorption characteristics of hemoglobin are low compared with the high absorption wavelength band A2 of 520 nm to 580 nm. Accordingly, when a captured image of image light of the second transmitted light is displayed on the monitor 14, the display of medium-deep layer blood vessels is suppressed, while the visibility of the hypertrophy having a thickness from a surface layer to a medium-deep layer is improved by suppressing the display of the blood vessels. As shown in Fig. 7C, third transmitted light of 580 nm to 780 nm, which is included in the R transmission region, of the high absorption wavelength cut light Wcut is incident on the R pixel of the CCD 44.

As shown in Fig. 2, the AFE 45 is configured to include a correlated double sampling circuit (CDS), an automatic gain control circuit (AGC), and an analog/digital converter (A/D) (all not shown). The CDS performs correlated double sampling processing on an imaging signal from the CCD 44 to remove noise caused by the driving of the CCD 44. The AGC amplifies an imaging signal from which noise has been removed by the CDS. The A/D converts an imaging signal amplified by the AGC into a digital imaging signal of a predetermined number of bits, and inputs the digital imaging signal to the processor device 12.

The imaging control unit 46 is connected to the controller 59 in the processor device 12, and transmits a driving signal to the CCD 44 when there is an instruction from the controller 59. The CCD 44 outputs an imaging signal to the AFE 45 at a predetermined frame rate based on the driving signal from the imaging control unit 46.

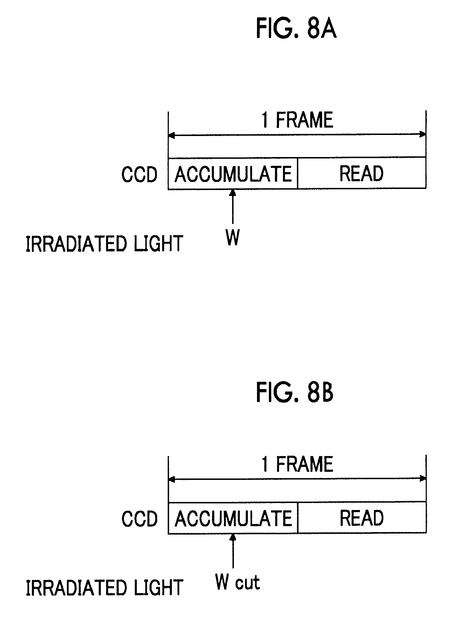

When the normal observation mode is set, as shown in Fig. 8A, a step of performing photoelectric conversion of image light of white light and accumulating the signal charges and a step of reading the accumulated signal charges are performed within one frame period. This imaging control is repeatedly performed. Signals that are output from the B, G, and R pixels of the CCD 44 in the normal observation mode are assumed to be a blue signal Bc, a green signal Gc, and a red signal Rc, respectively..

On the other hand, when the microstructure observation mode, the hypertrophy observation mode, and the microstructure and hypertrophy observation mode are set, as shown in Fig. 8B, a step of performing photoelectric conversion of image light of the high absorption wavelength cut light Wcut and accumulating the signal charges and a step of reading the accumulated signal charges are performed within one frame period. This imaging control is repeatedly performed. Signals that are output from the B, G, and R pixels of the CCD 44 in the microstructure observation mode, the hypertrophy observation mode, and the microstructure and hypertrophy observation mode are assumed to be a blue signal Bp, a green signal Gp, and a red signal Rp, respectively.

As shown in Fig. 2, the processor device 12 includes a normal light image generation unit 55, a frame memory 56, a special light image generation unit 57 (a form of irregularity image generation means), and a display control circuit 58. The controller 59 controls each of the units. The normal light image generation unit 55 generates a normal light image from the signals Bc, Gc, and Rc obtained in the normal observation mode. The generated normal light image is temporarily stored in the frame memory 56.

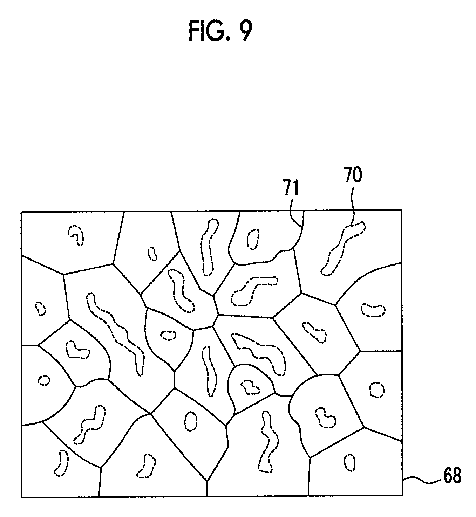

The special light image generation unit 57 includes a microstructure image generation section 61, a hypertrophy image generation section 62, and a microstructure and hypertrophy image generation section 63. The microstructure image generation section 61 generates a microstructure image, in which the visibility of a superficial microstructure, such as a pit pattern, is improved, based on the blue signal Bp obtained in the microstructure observation mode. The generated microstructure image 68 is displayed on the monitor 14 by the display control circuit 58, as shown in Fig. 9. Since the microstructure image 68 is generated using the high absorption wavelength cut light Wcut obtained by removing the high absorption wavelength band A1, the display of a superficial microvessel 70 is suppressed. Due to the suppression of this display, the visibility of a microstructure 71 is relatively improved.

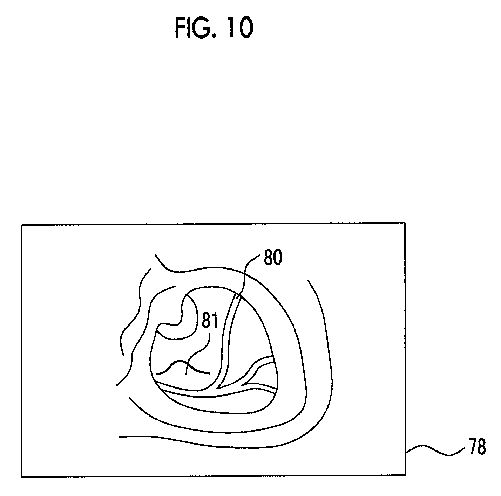

The hypertrophy image generation section 62 generates a hypertrophy image with improved visibility of hypertrophy based on the green signal Gp and the red signal Rp obtained in the hypertrophy observation mode. The generated hypertrophy image 78 is displayed on the monitor 14 by the display control circuit 58, as shown in Fig. 10. Since the hypertrophy image 78 is generated using the high absorption wavelength cut light Wcut obtained by removing the high absorption wavelength band A2, the display of a medium-deep layer blood vessel 80 is suppressed. Due to the suppression of this display, the visibility of a hypertrophy 81 is relatively improved.

The microstructure and hypertrophy image generation section 63 generates a microstructure and hypertrophy image with improved visibility of both a microstructure and hypertrophy based on the blue signal Bp, the green signal Gp, and the red signal Rp obtained in the microstructure and hypertrophy observation mode. The generated microstructure and hypertrophy image is displayed on the monitor 14 by the display control circuit 58.

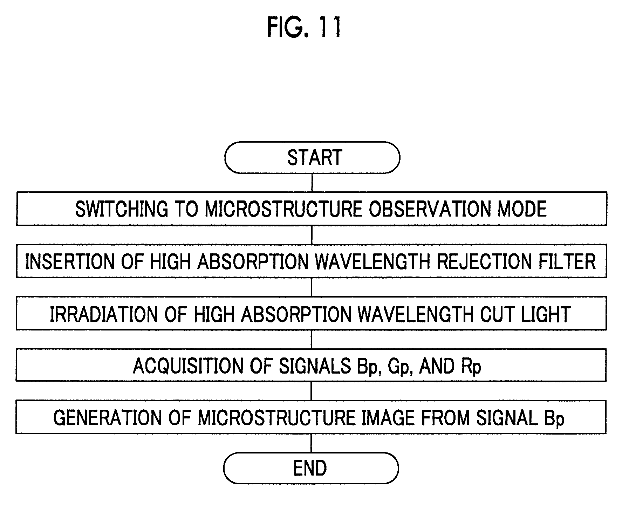

Next, a sequential flow in the microstructure observation mode will be described with reference to the flowchart shown in Fig. 11. In addition, a sequential flow in the hypertrophy observation mode and the microstructure observation mode is approximately the same, and accordingly, explanation thereof will be omitted.

When switching to the microstructure observation mode is performed by a mode switch SW 15, the high absorption wavelength rejection filter 33 is inserted in the optical path Lw of the white light W. Therefore, the high absorption wavelength cut light Wcut obtained by removing the wavelength components of the high absorption wavelength bands A1 and A2 from the white light W is emitted from the high absorption wavelength rejection filter 33. The transmitted high absorption wavelength cut light Wcut is irradiated to the subject through the condensing lens 34, the light guide 43, and the like.

Image light of the return light from the subject is captured by the color CCD 44. In this case, the blue signal Bp, the green signal Gp, and the red signal Rp are output from the B, G, and R pixels of the CCD 44, respectively. Based on the blue signal Bp of these signals, a microstructure image with improved visibility of the superficial microstructure is generated. The generated microstructure image is displayed on the monitor 14 by the display control circuit 58.

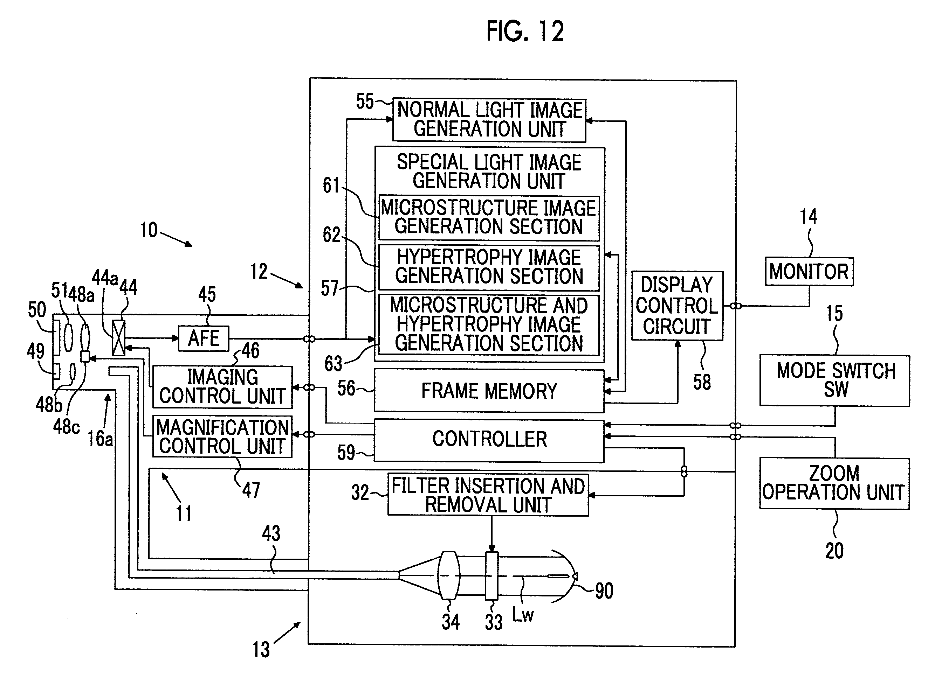

In the first embodiment, the white light W is generated by irradiating the phosphor 31 with the excitation light EL to excite and emit the fluorescence FL. Instead of this, however, as shown in Fig. 12, the white light W in a wavelength band of, for example, 380 nm to 700 nm may be generated using a xenon lamp 90. In addition, any light source that emits broadband light in a wavelength band of blue to red colors, such as a halogen lamp, can be used without being limited to the xenon lamp.

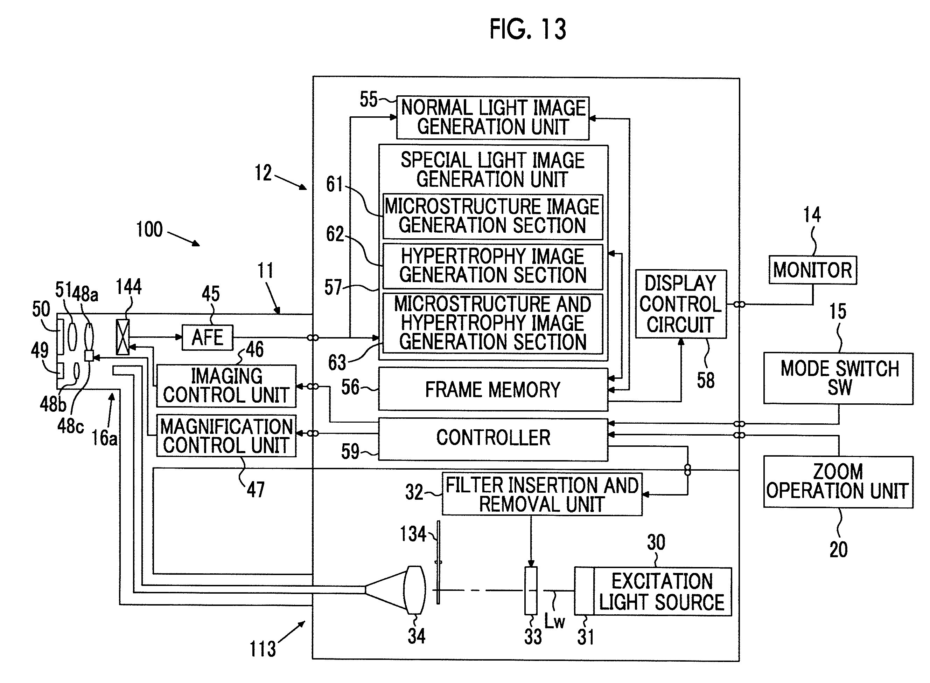

As shown in Fig. 13, an endoscope system 100 of a second embodiment performs subject imaging in a frame sequential method using a monochrome CCD 144, unlike in the first embodiment in which subject imaging is performed in a simultaneous system using the color CCD 44. Therefore, the configuration of a light source device 113 is different from that of the light source device 13 in the first embodiment. In addition, since the CCD in the electronic endoscope 11 is a monochrome imaging device 144 in which no color filter is provided, the imaging control method of the CCD 144 is also different from that in the first embodiment. Since others are the same as those described in the first embodiment, explanation thereof will be omitted.

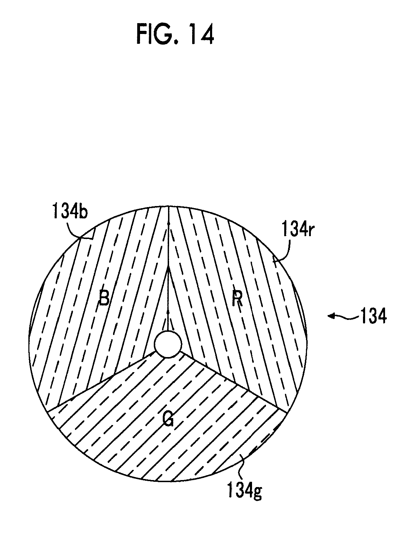

The light source device 113 includes an excitation light source 30, a phosphor 31, an RGB rotary filter 134, a filter insertion and removal unit 32, and a high absorption wavelength rejection filter 33. Also in the second embodiment, the white light W is generated by the excitation light source 30 and the phosphor 31. As shown in Fig. 14, the RGB rotary filter 134 has a disc shape, and is divided into three regions, each of which is a fan-shaped region having a central angle of 120°, in the circumferential direction, and a B filter portion 134b, a G filter portion 134g, and an R filter portion 134r are respectively provided in the three regions. The rotary filter 134 is rotatably provided so that the B filter portion 134a, the G filter portion 134b, and the R filter portion 134c are selectively inserted in the optical path Lw of the white light W.

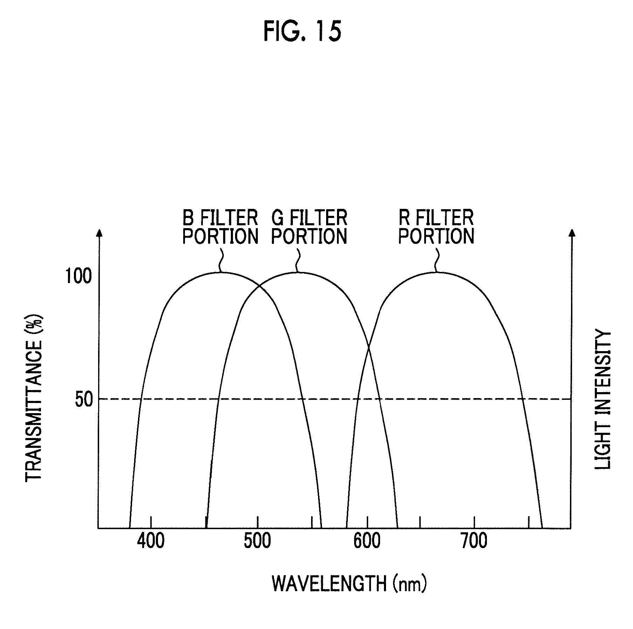

As shown in Fig. 15, the B filter portion 134b has the same B transmission region as the B filter 44b of the CCD 44 in the first embodiment. Similarly, the G filter portion 134g and the R filter portion 134r have the same G transmission region and R transmission region as the G filter 44g and the R filter 44r of the CCD 44, respectively.

As in the first embodiment, when the normal observation mode is set, the filter insertion and removal unit 32 sets the high absorption wavelength rejection filter 33 at the retraction position. Since the high absorption wavelength rejection filter 33 is set at the retraction position, the white light W from the phosphor 31 is incident on the RGB rotary filter 134 that is rotating, without being incident through the high absorption wavelength rejection filter 33. B light in the blue band of the white light W is transmitted when the B filter portion 134b of the RGB rotary filter 134 is inserted in the optical path Lw, G light in the green band of the white light W is transmitted when the G filter portion 134g of the RGB rotary filter 134 is inserted in the optical path Lw, and R light in the red band of the white light W is transmitted when the R filter portion 134r of the RGB rotary filter 134 is inserted in the optical path Lw. As a result, B light, G light, and R light are sequentially emitted from the RGB rotary filter 134. The B light, the G light, and the R light that are sequentially emitted are incident on the light guide 43 through the condensing lens 34 to irradiate the subject.

On the other hand, when the microstructure observation mode, the hypertrophy observation mode, and the microstructure and hypertrophy observation mode are set, the high absorption wavelength rejection filter 33 is set at the insertion position, as in the first embodiment. Since the high absorption wavelength rejection filter 33 is set at the insertion position, the white light W from the phosphor 31 is incident on the high absorption wavelength rejection filter 33. The high absorption wavelength rejection filter 33 is the same as that in the first embodiment, and transmits the high absorption wavelength cut light Wcut obtained by removing the wavelength components of the high absorption wavelength bands A1 and A2 from the white light W. The transmitted high absorption wavelength cut light Wcut is incident on the RGB rotary filter 134 that is rotating.

When the B filter portion 134b of the RGB rotary filter 134 is inserted in the optical path Lw, the first transmitted light included in the B transmission region of the high absorption wavelength cut light Wcut is transmitted. When the G filter portion 134g is inserted in the optical path Lw, the second transmitted light included in the G transmission region of the high absorption wavelength cut light Wcut is transmitted. When the R filter portion 134r is inserted in the optical path Lw, the third transmitted light included in the R transmission region of the high absorption wavelength cut light Wcut is transmitted. As a result, the first transmitted light, the second transmitted light, and the third transmitted light are sequentially emitted from the RGB rotary filter 134. The first transmitted light, the second transmitted light, and the third transmitted light that are sequentially emitted are incident on the light guide 43 through the condensing lens 34.

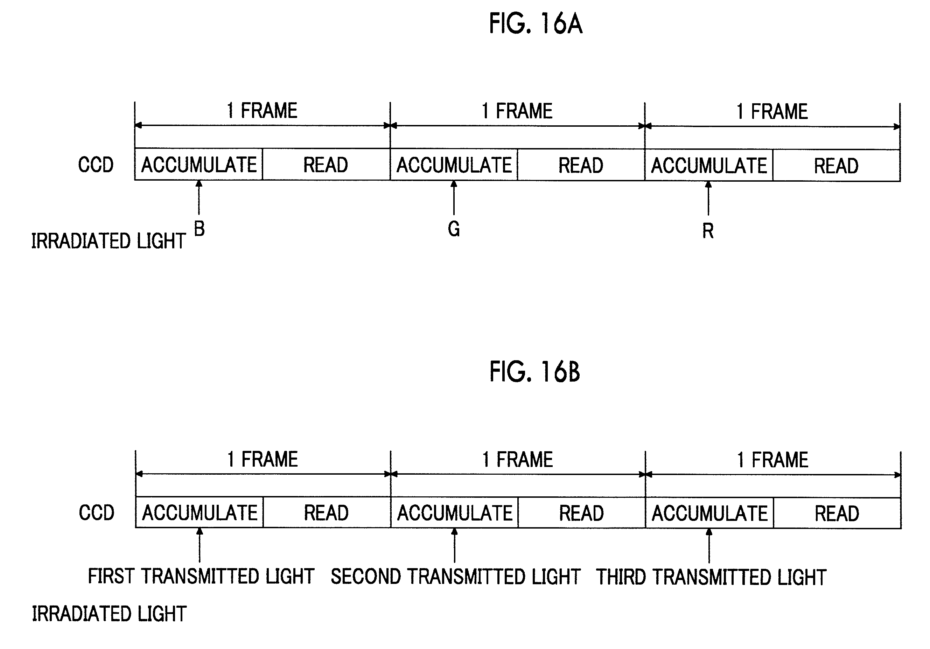

The imaging control unit 46 of the second embodiment controls the imaging of the monochrome CCD 144 as follows. In the normal observation mode, as shown in Fig. 16A, image light beams of three colors of B, G, and R are sequentially captured and the electric charges are accumulated, and frame sequential imaging signals B, G, and R are sequentially output based on the accumulated electric charges. The series of operations are repeated while the normal observation mode is set. The frame sequential imaging signals B, G, and R approximately correspond to Bc, Gc, and Rc of the first embodiment, respectively.

In the microstructure observation mode, the hypertrophy observation mode, and the microstructure and hypertrophy observation mode, as shown in Fig. 16B, image light beams of the first transmitted light, the second transmitted light, and the third transmitted light are sequentially captured and the electric charges are accumulated, and frame sequential imaging signals X1, X2, and X3 are sequentially output based on the accumulated electric charges. The series of operations are repeated while the microstructure observation mode, the hypertrophy observation mode, and the microstructure and hypertrophy observation mode are set. The frame sequential imaging signals X1, X2, and X3 correspond to Bp, Gp, and Rp of the first embodiment, respectively.

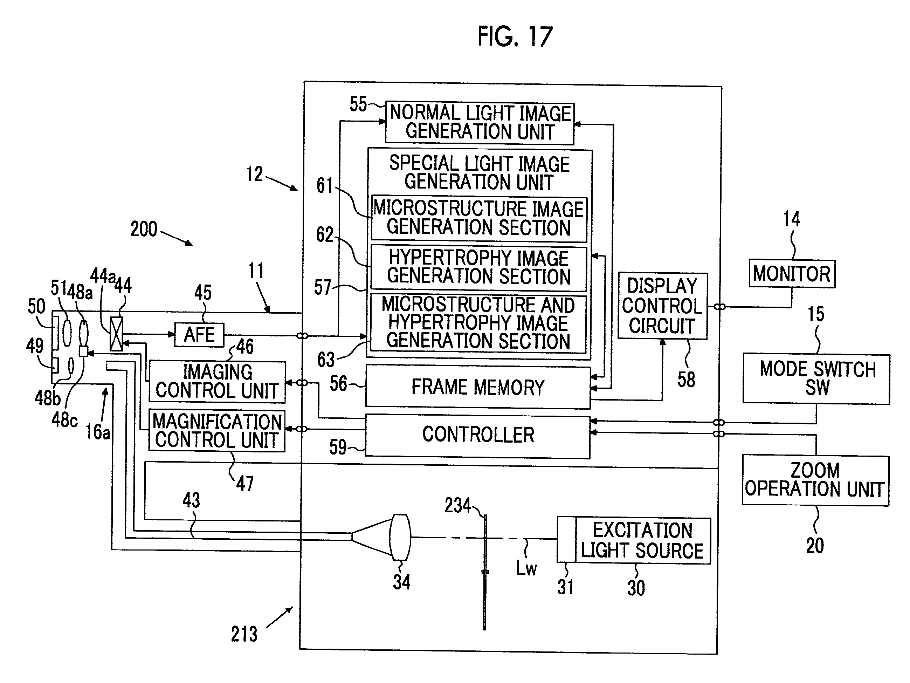

As shown in Fig. 17, in an endoscope system 200 of a third embodiment, a rotary filter for special observation 234 is used to generate the high absorption wavelength cut light Wcut, unlike in the first and second embodiments in which the high absorption wavelength rejection filter 33 that can be freely inserted into or removed from the optical path Lw of the white light W is used. Due to the use of the rotary filter for special observation 234, the imaging control method of the color CCD 44 is different from that in the first embodiment. In addition, the image generation method of a microstructure image, a hypertrophy image, and a microstructure and hypertrophy image is different from that in the first embodiment. Since others are the same as those described in the first embodiment, explanation thereof will be omitted.

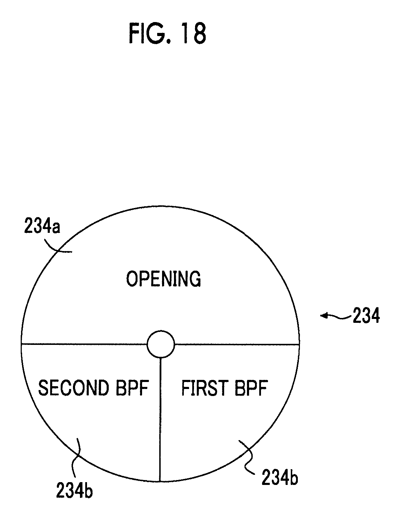

As shown in Fig. 18, in the rotary filter for special observation 234, an opening 234a that transmits the white light W as it is, a first band pass filter (BPF) 234b that transmits illumination light for a surface layer, which is used to improve the visibility of the superficial microstructure, of the white light W, and a second band pass filter (BPF) 234c that transmits illumination light for a medium-deep layer, which is used to improve the visibility of the hypertrophy, of the white light W are provided along the circumferential direction. The rotary filter for special observation 234 is rotatably provided so that the opening 234a, the first BPF 234b, and the second BPF 234c are selectively inserted in the optical path Lw of the white light W.

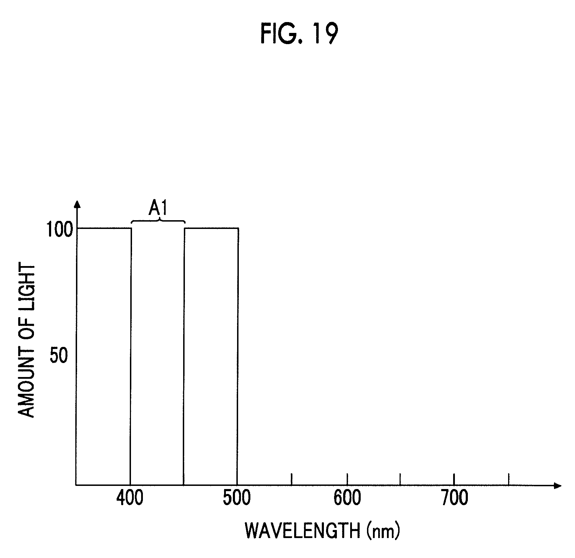

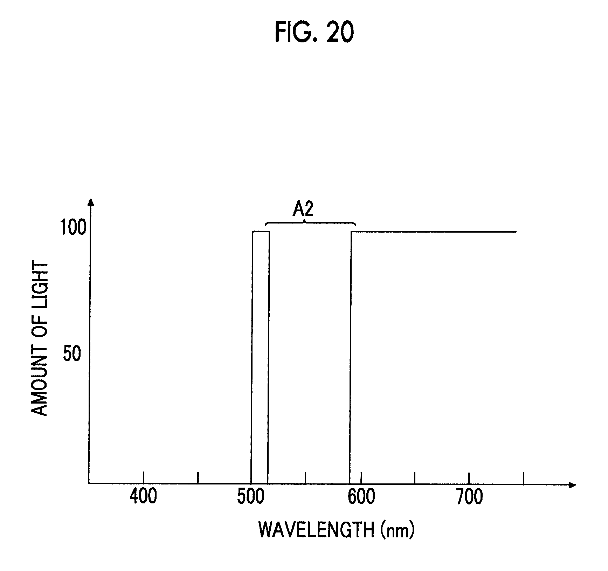

As shown in Fig. 19, the first BPF 234b cuts light in the high absorption wavelength band A1 of 400 nm to 450 nm and light in a wavelength band of 500 nm or more of the blue component of the white light W (with a transmittance of 0%), and transmits light of 400 nm or less and light of 450 nm to 500 nm (transmittance of 100%). Accordingly, the illumination light for a surface layer obtained after the white light W is transmitted through the first BPF 234b becomes light having a wavelength band of 400 nm or less and 450 nm to 500 nm. On the other hand, as shown in Fig. 20, the second BPF 234c cuts light in the high absorption wavelength band A2 of 520 nm to 580 nm and light in a wavelength band of 500 nm or less of the green and red components of the white light W, and transmits light in a wavelength band of 500 nm to 520 nm and light in a wavelength band of 580 nm or more. Accordingly, the illumination light for a medium-deep layer obtained after the white light W is transmitted through the second BPF 234c becomes light having a wavelength band of 500 nm to 520 nm and a wavelength band of 580 nm or more.

The white light W is transmitted as it is when the opening 234a of the rotary filter for special observation 234 is inserted in the optical path Lw, the illumination light for a surface layer of the white light W is transmitted when the first BPF 234b is inserted in the optical path Lw, and the illumination light for a medium-deep layer of the white light W is transmitted when the second BPF 234c is inserted in the optical path Lw. Therefore, the white light W, the illumination light for a surface layer, and the illumination light for a medium-deep layer are sequentially emitted from the rotary filter for special observation 234. The white light W, the illumination light for a surface layer, and the illumination light for a medium-deep layer that are sequentially emitted are incident on the light guide 43 through the condensing lens 34.

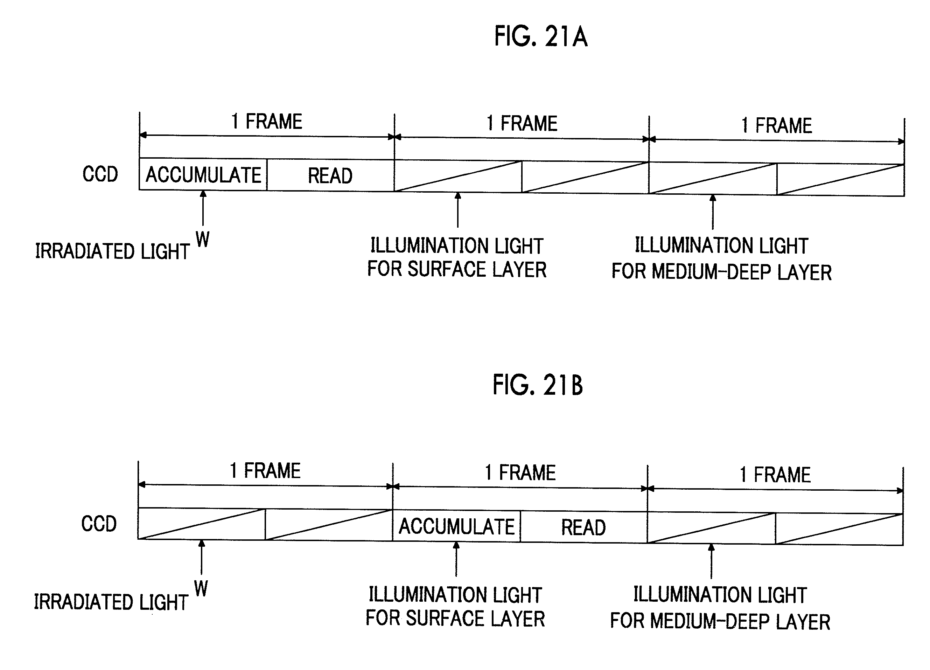

The imaging control unit 46 of the third embodiment controls the imaging of the color CCD 44 as follows. In the normal observation mode, as shown in Fig. 21A, image light of the white light W is captured and the electric charges are accumulated, and imaging signals B1, G1, and R1 are output from the B, G, and R pixels of the CCD 44 based on the accumulated electric charges. On the other hand, when the illumination light for a surface layer and the illumination light for a medium-deep layer are irradiated, the accumulation of electric charges and the output of imaging signals are not performed. The series of operations are repeated while the normal observation mode is set. The imaging signals B1, G1, and R1 correspond to Bc, Gc, and Rc of the first embodiment, respectively.

In the microstructure observation mode, as shown in Fig. 21B, when the white light W and the illumination light for a medium-deep layer are irradiated, the accumulation of electric charges and the output of imaging signals are not performed. On the other hand, when the illumination light for a surface layer is irradiated, image light beams of these light beams are sequentially captured and the electric charges are accumulated. Then, imaging signals B2, G2, and R2 are output from the B, G, and R pixels of the CCD 44 based on the accumulated electric charges. The series of operations are repeated while the microstructure observation mode is set. The imaging signal B2 corresponds to Bp of the first embodiment.

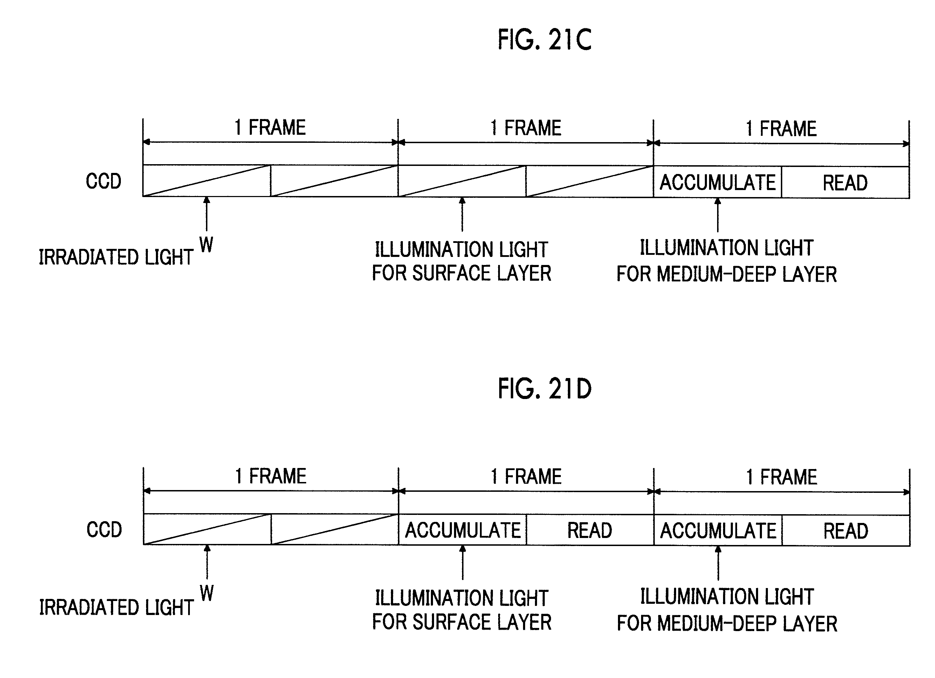

In the hypertrophy observation mode, as shown in Fig. 21C, when the white light W and the illumination light for a surface layer are irradiated, the accumulation of electric charges and the output of imaging signals are not performed. On the other hand, when the illumination light for a medium-deep layer is irradiated, image light beams of these light beams are sequentially captured and the electric charges are accumulated. Then, imaging signals B3, G3, and R3 are output from the B, G, and R pixels of the CCD 44 based on the accumulated electric charges. The series of operations are repeated while the hypertrophy observation mode is set. The imaging signals G3 and R3 correspond to Gp and Rp of the first embodiment.

In the microstructure and hypertrophy observation mode, as shown in Fig. 21D, when the white light W is irradiated, the accumulation of electric charges and the output of imaging signals are not performed. On the other hand, when the illumination light for a surface layer and the illumination light for a medium-deep layer are irradiated, image light beams of these light beams are sequentially captured and the electric charges are accumulated. Then, based on the accumulated electric charges, the imaging signals B2, G2, and R2 are output from the B, G, and R pixels of the CCD 44 when the illumination light for a surface layer is irradiated, and the imaging signals B3, G3, and R3 are output from the B, G, and R pixels when the illumination light for a medium-deep layer is irradiated. The series of operations are repeated while the microstructure and hypertrophy observation mode is set. In the same manner as described above, the imaging signal B2 approximately corresponds to Bp of the first embodiment, and the imaging signals G3 and R3 correspond to Gp and Rp of the second embodiment.

In the microstructure observation mode, the hypertrophy observation mode, and the microstructure and hypertrophy observation mode, the accumulation of electric charges and the output of imaging signals may also be performed when the white light W is irradiated, so that a normal light image based on the imaging signal obtained from the output is generated. By adding the pixel value of the imaging signal B2 to the normal light image, a brighter microstructure image can be generated. In addition, by adding the pixel values of the imaging signals G3 and R3 to the normal light image, a brighter hypertrophy image can be generated.

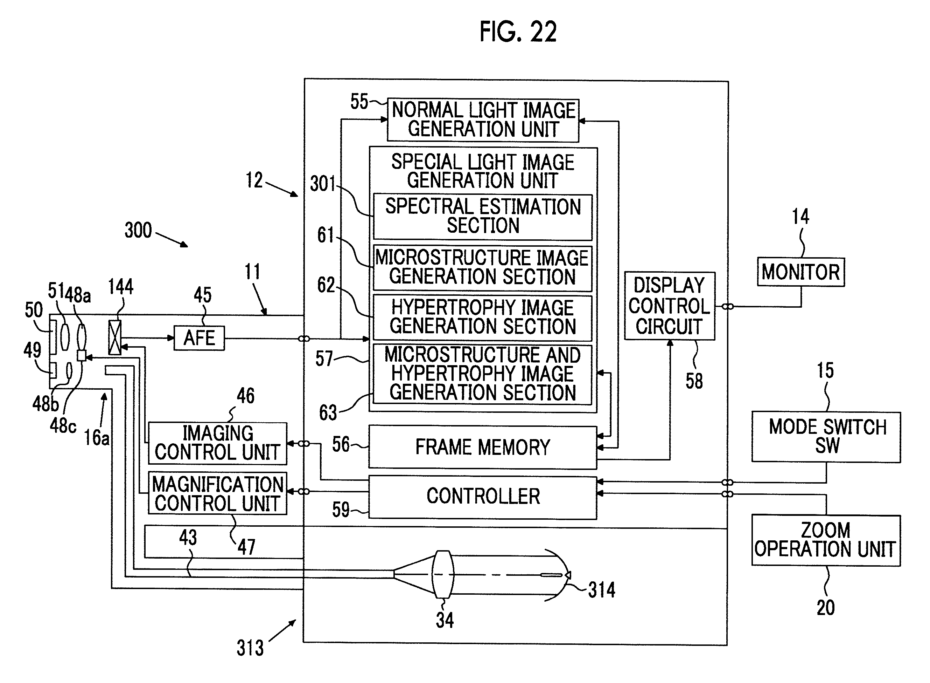

As shown in Fig. 22, in an endoscope system 300 of a fourth embodiment, required wavelength components are acquired using a spectral estimation technique, unlike in the first and second embodiments in which wavelength components required to generate a microstructure image or a hypertrophy image are acquired by wavelength separation using the high absorption wavelength rejection filter. Therefore, the high absorption wavelength rejection filter 33 and the filter insertion and removal unit 32 for inserting or removing the high absorption wavelength rejection filter 33 into or from the optical path Lw of the white light W is not provided in the light source device 13 of the endoscope system 300. In addition, the image generation method of a microstructure image, a hypertrophy image, and a microstructure and hypertrophy image is different from that in the first embodiment. Since others are the same as those described in the first embodiment, explanation thereof will be omitted.

In a light source device 313 of the fourth embodiment, unlike the first embodiment, the white light W having a wavelength band of 380 nm to 700 nm is generated by a xenon lamp 314. The xenon lamp 314 is always lit. Accordingly, the white light W emitted from the xenon lamp 314 is always irradiated to the subject through the condensing lens 34 and the light guide 43. Then, similarly to the first embodiment, image light of the white light from the subject is captured by the color CCD 44. By this imaging, the blue signal B is output from the B pixel of the CCD 44, the green signal G is output from the G pixel, and the red signal R is output from the R pixel.

A spectral estimation section 301 in the special light image generation unit 57 generates a spectral image within 380 nm to 700 nm based on the signals B, G, and R. Spectral images are generated at intervals of 5 nm as a 380-nm image, a 385-nm image, and the like. The spectral estimation section 301 performs spectral estimation according to the following [Expression 1] using estimation matrix data stored in an internal memory (not shown).

[Expression 1]

$$\begin{bmatrix} q_1 \\ q_2 \\ \vdots \\ q_{65} \end{bmatrix} = \begin{bmatrix} k_{1r} & k_{1g} & k_{1b} \\ k_{2r} & k_{2g} & k_{2r} \\ \vdots & \vdots & \vdots \\ k_{65r} & k_{65g} & k_{65b} \end{bmatrix} \times \begin{bmatrix} R \\ G \\ B \end{bmatrix}$$

In [Expression 1], pixel values of the signals B, G, and R are expressed as B, G, and R, respectively. In addition, the estimation matrix data is configured to include 65 sets of wavelength band parameters obtained by dividing the wavelength band of 380 nm to 700 nm at intervals of 5 nm, and the respective wavelength band parameters are formed by coefficients knr, kng, and knb (n = 1 to 65). By multiplying these wavelength band parameters by the pixel values of the signals B, G, and R, pixel values qn (n = 1 to 65) of spectral images from 380 nm to 700 nm are obtained. In addition, the method of generating a spectral image is disclosed in detail in JP2003-93336A.

When the microstructure observation mode is set, the spectral estimation section 301 acquires a spectral image of 380 nm to 400 nm and a spectral image of 450 nm to 500 nm. In addition, when the hypertrophy observation mode is set, a spectral image of 500 nm to 520 nm and a spectral image of 580 nm to 700 nm are acquired. When the microstructure and hypertrophy observation mode is set, a spectral image of 380 nm to 400 nm, a spectral image of 450 nm to 500 nm, a spectral image of 500 nm to 520 nm, and a spectral image of 580 nm to 700 nm are acquired.

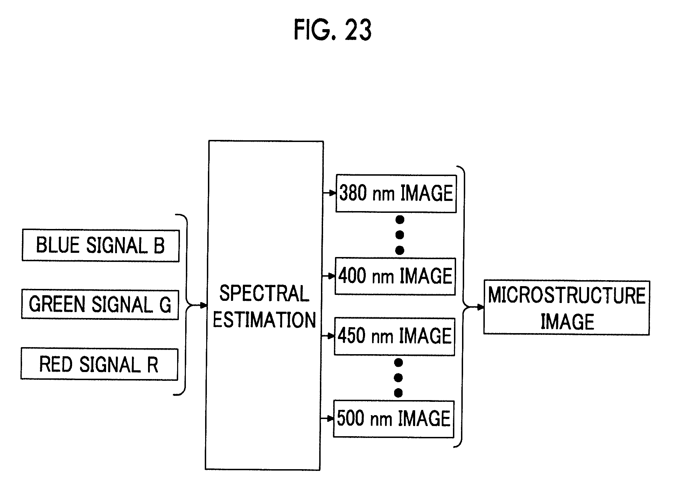

As shown in Fig. 23, the microstructure image generation section 61 of the fourth embodiment generates a microstructure image based on the spectral image of 380 nm to 400 nm and the spectral image of 450 nm to 500 nm obtained by the spectral estimation section 301. This microstructure image has the same wavelength components as in the microstructure image of the first embodiment. Therefore, since the display of superficial microvessels is suppressed as in the first embodiment, the visibility of the microstructure is relatively improved.

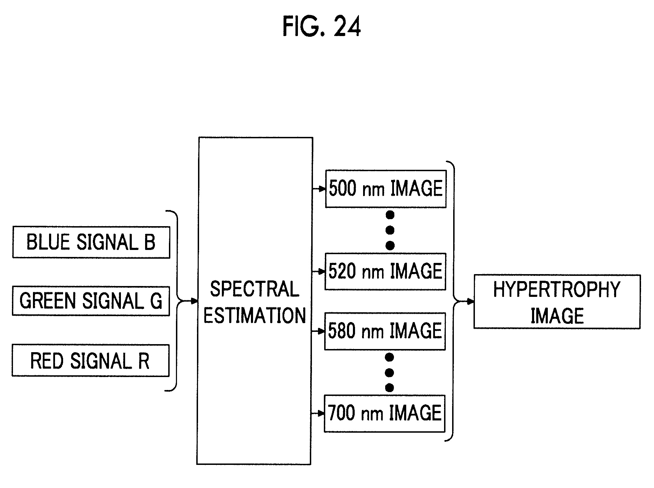

As shown in Fig. 24, the hypertrophy image generation section 62 of the fourth embodiment generates a hypertrophy image based on the spectral image of 500 nm to 520 nm and the spectral image of 580 nm to 700 nm obtained by the spectral estimation section 301. This hypertrophy image also has the same wavelength components as in the hypertrophy image of the first embodiment. Therefore, since the display of medium-deep layer blood vessels is suppressed as in the first embodiment, the visibility of hypertrophy is relatively improved.

The microstructure and hypertrophy generation section 63 of the fourth embodiment generates a microstructure and hypertrophy image based on the spectral image of 380 nm to 400 nm, the spectral image of 450 nm to 500 nm, the spectral image of 500 nm to 520 nm, and the spectral image of 580 nm to 700 nm obtained by the spectral estimation section 301. This microstructure and hypertrophy image also has the same wavelength components as in the microstructure and hypertrophy image of the first embodiment. Therefore, as in the first embodiment, the visibility of both the microstructure and the hypertrophy is improved.

**Claims**

1. An endoscope system, comprising:

    an illumination means for irradiating a subject with illumination light;

an image signal acquisition means for acquiring an image signal by capturing image light of reflected light from the subject; and
an irregularity image generation means for generating an irregularity image, in which visibility of irregularities on body tissue of the subject is relatively improved, by suppressing display of blood vessels in the subject based on the image signal.

2. The endoscope system according to claim 1, wherein the irregularity image generation means includes a microstructure image generation section that generates a microstructure image, in which visibility of superficial microstructures is relatively improved, by suppressing display of blood vessels based on a signal obtained by removing a component of a first high absorption wavelength band, in which an absorption coefficient of hemoglobin in blood is high, in a blue wavelength band from the image signal.

3. The endoscope system according to claim 2, wherein the first high absorption wavelength band is 400 nm to 450 nm.

4. The endoscope system according to any one of claims 1 to 3, wherein the irregularity image generation means includes a microstructure image generation section that generates a microstructure image, in which visibility of hypertrophy is relatively improved, by suppressing display of blood vessels based on a signal obtained by removing a component of a second high absorption wavelength band, in which an absorption coefficient of hemoglobin in blood is high, in a green wavelength band from the image signal.

5. The endoscope system according to claim 4, wherein the second high absorption wavelength band is 520 nm to 580 nm.

6. The endoscope system according to any one of claims 1 to 5, wherein the illumination means includes a high absorption wavelength rejection filter that removes a component of a high absorption wavelength band, in which an absorption coefficient of hemoglobin in blood is high, from the illumination light, and the image signal acquisition means captures image light of reflected light from the subject, from which the component of the high absorption wavelength band has been removed by the high absorption wavelength rejection filter.

7. The endoscope system according to claim 6, wherein imaging of the subject is performed by a color imaging device having pixels of a plurality of colors for which respective color separation filters are provided.

8. The endoscope system according to claim 6, wherein the illumination means irradiates the subject sequentially with light beams of a plurality of colors, and
imaging of the subject is performed by a monochrome imaging device whenever the light beams of the plurality of colors are sequentially irradiated to the subject.

9. The endoscope system according to claim 4 or 5, wherein the illumination means sequentially irradiates illumination light for a surface layer obtained by removing a component of a first high absorption wavelength band, in which an absorption coefficient of hemoglobin in blood is high, in a blue wavelength band and illumination light for a medium-deep layer obtained by removing a component of a first high absorption wavelength band, in which an absorption coefficient of hemoglobin in blood is high, in a green wavelength band, and
imaging of the subject is performed whenever sequential irradiation is performed by the illumination means.

10. The endoscope system according to any one of claims 1 to 5,
wherein the irregularity image generation means includes an image generation section that acquires a spectral image of a wavelength component other than a wavelength component, in which an absorption coefficient of hemoglobin in blood is high, of the reflected light by spectral estimation based on the image signal and generates the irregularity image based on the spectral image.

11. The endoscope system according to any one of claims 1 to 10, further comprising:

display means for displaying the irregularity image.

12. An image generation method, comprising:

irradiating a subject with illumination light using illumination means;
acquiring an image signal by capturing image light of reflected light from the subject using image signal acquisition means; and
generating an irregularity image, in which visibility of irregularities on body tissue is relatively improved, by suppressing display of blood vessels in the subject based on the image signal using irregularity image generation means.

**Amended claims under Art. 19.1 PCT**

1. (Cancelled)

2. An endoscope system, comprising:

an illumination means for irradiating a subject with illumination light;
an image signal acquisition means for acquiring an image signal by capturing image light of reflected light from the subject;
an irregularity image generation means for generating an irregularity image, in which visibility of irregularities on body tissue of the subject is relatively improved, by suppressing display of blood vessels in the subject based on the image signal; and
wherein the irregularity image generation means includes a microstructure image generation section that generates a microstructure image, in which visibility of superficial microstructures is relatively improved, by suppressing display of blood vessels based on a signal obtained by removing a component of a first high absorption wavelength band, in which an absorption coefficient of hemoglobin in blood is high, in a blue wavelength band from the image signal.

3. The endoscope system according to claim 2, wherein the first high absorption wavelength band is 400 nm to 450 nm.

4. The endoscope system according to claim 2 or 3, wherein the irregularity image generation means includes a microstructure image generation section that generates a microstructure image, in which visibility of hypertrophy is relatively improved, by suppressing display of blood vessels based on a signal obtained by removing a component of a second high absorption wavelength band, in which an absorption coefficient of hemoglobin in blood is high, in a green wavelength band from the image signal.

5. The endoscope system according to claim 4, wherein the second high absorption wavelength band is 520 nm to 580 nm.

6. The endoscope system according to any one of claims 2 to 5,
wherein the illumination means includes a high absorption wavelength rejection filter that removes a component of a high absorption wavelength band, in which an absorption coefficient of hemoglobin in blood is high, from the illumination light, and
the image signal acquisition means captures image light of reflected light from the subject, from which the component of the high absorption wavelength band has been removed by the high absorption wavelength rejection filter.

**7.** The endoscope system according to claim 6, wherein imaging of the subject is performed by a color imaging device having pixels of a plurality of colors for which respective color separation filters are provided.

**8.** The endoscope system according to claim 6, wherein the illumination means irradiates the subject sequentially with light beams of a plurality of colors, and
imaging of the subject is performed by a monochrome imaging device whenever the light beams of the plurality of colors are sequentially irradiated to the subject.

**9.** The endoscope system according to claim 4 or 5, wherein the illumination means sequentially irradiates illumination light for a surface layer obtained by removing a component of a first high absorption wavelength band, in which an absorption coefficient of hemoglobin in blood is high, in a blue wavelength band and illumination light for a medium-deep layer obtained by removing a component of a first high absorption wavelength band, in which an absorption coefficient of hemoglobin in blood is high, in a green wavelength band, and
imaging of the subject is performed whenever sequential irradiation is performed by the illumination means.

**10.** The endoscope system according to any one of claims 2 to 5, wherein the irregularity image generation means includes an image generation section that acquires a spectral image of a wavelength component other than a wavelength component, in which an absorption coefficient of hemoglobin in blood is high, of the reflected light by spectral estimation based on the image signal and generates the irregularity image based on the spectral image.

**11.** The endoscope system according to any one of claims 2 to 10, further comprising:

display means for displaying the irregularity image.

**12.** An image generation method, comprising:

irradiating a subject with illumination light using illumination means;
acquiring an image signal by capturing image light of reflected light from the subject using image signal acquisition means;
generating an irregularity image, in which visibility of irregularities on body tissue is relatively improved, by suppressing display of blood vessels in the subject based on the image signal using irregularity image generation means; and
wherein the irregularity image generation means includes a microstructure image generation section that generates a microstructure image, in which visibility of superficial microstructures is relatively improved, by suppressing display of blood vessels based on a signal obtained by removing a component of a first high absorption wavelength band, in which an absorption coefficient of hemoglobin in blood is high, in a blue wavelength band from the image signal.

**Statement under Art. 19.1 PCT**

1. Content of Amendment

(1) Applicant amends claim 2 as an independent claim by incorporating claim element of claim 1 to claim 2.
(2) Dependency of Claim 4 is amended from "claims 1 to 3" to "claim 2 or 3".
(4) Dependency of Claim 6 is amended from "claims 1 to 5" to "claims 2 to 5".
(5) Dependency of Claim 10 is amended from "claims 1 to 5" to "claims 2 to 5".
(6) Dependency of Claim 11 is amended from "claims 1 to 10" to "claims 2 to 10".
(7) Claim 12 is amended from method claim of original claim 1 to method claim of amended claim 2.
(8) Claim 1 is canceled.

2. Explanation

Claim element described in claim 1 is incorporated to claim 2, and claim 2 is amended as an independent claim. Also, according to the amendment mentioned above, dependency of claim 4 is amended from "claims 1 to 3" to "claim 2 or 3". Further, dependency of Claim 6 is amended from "claims 1 to 5" to "claims 2 to 5". In addition, dependency of Claim 10 is amended from "claims 1 to 5" to "claims 2 to 5". Furthermore, dependency of Claim 11 is amended from "claims 1 to 10" to "claims 2 to 10". Further, according to the amendment shown above, claim 1 is canceled.

In addition, claim 12 is amended from method claim of original claim 1 to method claim of amended claim 2, and is amended not to be regarded as a method for treatment of the human or animal body by surgery. Basis of such amendment is described in paragraphs [0022] to [0043]. In the endoscope system and image generation method stated by claims 1 to 12, claim elements described above is not disclosed in any of cited documents.

FIG. 1

EP 2 767 210 A1

# FIG. 2

EP 2 767 210 A1

FIG. 3

# FIG. 4

## FIG. 5A

## FIG. 5B

FIG. 6A

44b

44g

B

G

R ~ 44r

FIG. 6B

FIG. 7A

# FIG. 7B

FIG. 7C

## FIG. 8A

1 FRAME

CCD | ACCUMULATE | READ

IRRADIATED LIGHT    W

## FIG. 8B

1 FRAME

CCD | ACCUMULATE | READ

IRRADIATED LIGHT    W cut

FIG. 9

# FIG. 10

FIG. 11

```
              ┌─────────────────────┐
              │        START        │
              └─────────────────────┘
                         │
┌───────────────────────────────────────────────────────┐
│     SWITCHING TO MICROSTRUCTURE OBSERVATION MODE        │
└───────────────────────────────────────────────────────┘
                         │
┌───────────────────────────────────────────────────────┐
│  INSERTION OF HIGH ABSORPTION WAVELENGTH REJECTION FILTER │
└───────────────────────────────────────────────────────┘
                         │
┌───────────────────────────────────────────────────────┐
│   IRRADIATION OF HIGH ABSORPTION WAVELENGTH CUT LIGHT   │
└───────────────────────────────────────────────────────┘
                         │
┌───────────────────────────────────────────────────────┐
│        ACQUISITION OF SIGNALS Bp, Gp, AND Rp            │
└───────────────────────────────────────────────────────┘
                         │
┌───────────────────────────────────────────────────────┐
│   GENERATION OF MICROSTRUCTURE IMAGE FROM SIGNAL Bp     │
└───────────────────────────────────────────────────────┘
                         │
              ┌─────────────────────┐
              │         END         │
              └─────────────────────┘
```

# FIG. 12

55 — NORMAL LIGHT IMAGE GENERATION UNIT

SPECIAL LIGHT IMAGE GENERATION UNIT

61 — MICROSTRUCTURE IMAGE GENERATION SECTION

62 — HYPERTROPHY IMAGE GENERATION SECTION

57 — MICROSTRUCTURE AND HYPERTROPHY IMAGE GENERATION SECTION

63

DISPLAY CONTROL CIRCUIT

58

14 — MONITOR

15

MODE SWITCH SW

20 — ZOOM OPERATION UNIT

AFE — 45

46 — IMAGING CONTROL UNIT

56 — FRAME MEMORY

47 — MAGNIFICATION CONTROL UNIT

59 — CONTROLLER

32 — FILTER INSERTION AND REMOVAL UNIT

50, 5148a, 44a44, 49, 48b, 48c, 16a, 11, 43, 34, 33, Lw, 90

10, 12, 13

EP 2 767 210 A1

FIG. 13

EP 2 767 210 A1

FIG. 14

# FIG. 15

# FIG. 16A

| | ACCUMULATE | READ | ACCUMULATE | READ | ACCUMULATE | READ |
|---|---|---|---|---|---|---|
| CCD | | | | | | |

| 1 FRAME | 1 FRAME | 1 FRAME |
|---|---|---|

IRRADIATED LIGHT: B → G → R

# FIG. 16B

| | ACCUMULATE | READ | ACCUMULATE | READ | ACCUMULATE | READ |
|---|---|---|---|---|---|---|
| CCD | | | | | | |

| 1 FRAME | 1 FRAME | 1 FRAME |
|---|---|---|

FIRST TRANSMITTED LIGHT    SECOND TRANSMITTED LIGHT    THIRD TRANSMITTED LIGHT

IRRADIATED LIGHT

FIG. 17

EP 2 767 210 A1

FIG. 18

FIG. 19

# FIG. 20

## FIG. 21A

| 1 FRAME | | 1 FRAME | 1 FRAME |
|---|---|---|---|

CCD

| ACCUMULATE | READ | | |

IRRADIATED LIGHT W

ILLUMINATION LIGHT
FOR SURFACE LAYER

ILLUMINATION LIGHT
FOR MEDIUM-DEEP LAYER

## FIG. 21B

| 1 FRAME | 1 FRAME | 1 FRAME |
|---|---|---|

CCD

| | ACCUMULATE | READ | |

IRRADIATED LIGHT W

ILLUMINATION LIGHT
FOR SURFACE LAYER

ILLUMINATION LIGHT
FOR MEDIUM-DEEP LAYER

EP 2 767 210 A1

# FIG. 21C

| | 1 FRAME | | 1 FRAME | | 1 FRAME | |
|---|---|---|---|---|---|---|
| CCD | | | | | ACCUMULATE | READ |

↑ IRRADIATED LIGHT W

↑ ILLUMINATION LIGHT FOR SURFACE LAYER

↑ ILLUMINATION LIGHT FOR MEDIUM-DEEP LAYER

# FIG. 21D

| | 1 FRAME | | 1 FRAME | | 1 FRAME | |
|---|---|---|---|---|---|---|
| CCD | | | ACCUMULATE | READ | ACCUMULATE | READ |

↑ IRRADIATED LIGHT W

↑ ILLUMINATION LIGHT FOR SURFACE LAYER

↑ ILLUMINATION LIGHT FOR MEDIUM-DEEP LAYER

EP 2 767 210 A1

FIG. 22

EP 2 767 210 A1

# FIG. 23

FIG. 24

# FIG. 25

BLUE SIGNAL B

GREEN SIGNAL G

RED SIGNAL R

SPECTRAL ESTIMATION

380 nm IMAGE

400 nm IMAGE

450 nm IMAGE

500 nm IMAGE

500 nm IMAGE

520 nm IMAGE

580 nm IMAGE

700 nm IMAGE

MICROSTRUCTURE AND HYPERTROPHY IMAGE

## INTERNATIONAL SEARCH REPORT

| | International application No. |
|---|---|
| | PCT/JP2012/076291 |

### A. CLASSIFICATION OF SUBJECT MATTER
*A61B1/00*(2006.01)i

According to International Patent Classification (IPC) or to both national classification and IPC

### B. FIELDS SEARCHED

Minimum documentation searched (classification system followed by classification symbols)
A61B1/00

Documentation searched other than minimum documentation to the extent that such documents are included in the fields searched

| Jitsuyo Shinan Koho | 1922-1996 | Jitsuyo Shinan Toroku Koho | 1996-2012 |
|---|---|---|---|
| Kokai Jitsuyo Shinan Koho | 1971-2012 | Toroku Jitsuyo Shinan Koho | 1994-2012 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2008-183349 A (Olympus Corp.), 14 August 2008 (14.08.2008), paragraphs [0011], [0031] to [0036]; fig. 4 to 9 & US 2010/0103250 A1 & EP 2108300 A1 & WO 2008/093746 A1 | 1,11 |
| X | JP 2011-98088 A (Fujifilm Corp.), 19 May 2011 (19.05.2011), paragraphs [0007], [0046], [0048] & US 2011/0112362 A1 & EP 2328339 A2 | 1,11 |

☐ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 20 December, 2012 (20.12.12) | 08 January, 2013 (08.01.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

# EP 2 767 210 A1

<table>
<tr><td style="text-align:center">INTERNATIONAL SEARCH REPORT</td><td>International application No.<br>PCT/JP2012/076291</td></tr>
</table>

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 12
   because they relate to subject matter not required to be searched by this Authority, namely:
   (See extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

42

**INTERNATIONAL SEARCH REPORT**

International application No.

PCT/JP2012/076291

Continuation of Box No.II-1 of continuation of first sheet(2)

The method specified in claim 12 involves a surgical treatment, i.e., the insertion of an endoscope. Therefore, this method pertains to a method for the treatment of a human or animal body by surgery, and thus relates to a subject matter which this international searching authority is not required, under the provisions of PCT Article 17(2)(a)(i) and PCT Rule 39.1(iv), to search.

Form PCT/ISA/210 (extra sheet) (July 2009)

## REFERENCES CITED IN THE DESCRIPTION

**Patent documents cited in the description**

- JP 2001170009 A
- JP 8252218 A
- JP H08252218 A
- JP 2003093336 A